# EUROPEAN PATENT APPLICATION

(11) **EP 4 786 598 A1**
(43) Date of publication of application: **05.08.2026**
(21) Application number: 24868338.5
(22) Date of filing: 20.09.2024
(51) Int. Cl.: C12N 15/49, A61K 31/7088, A61K 31/7115, A61P 31/18, C12N 15/12, C12N 15/62

(54) **POLYNUCLEOTIDE ENCODING VIRUS-DERIVED PROTEIN**

(30) Priority: 22.09.2023 JP 2023158238
(71) Applicant: Shionogi & Co., Ltd., Osaka-shi, Osaka 541-0045 (JP)
(72) Inventor: YOSHINAGA, Tomokazu, Osaka-shi, Osaka 541-0045 (JP); MITSUOKA, Yasunori, Osaka-shi, Osaka 541-0045 (JP); KITAHATA, Shun, Osaka-shi, Osaka 541-0045 (JP); KURODA, Norikazu, Osaka-shi, Osaka 541-0045 (JP); KUGIMIYA, Akira, Osaka-shi, Osaka 541-0045 (JP); TAKIYAMA, Kei, Osaka-shi, Osaka 541-0045 (JP); MAEDA, Kousuke, Osaka-shi, Osaka 541-0045 (JP); TANINO, Tetsuya, Osaka-shi, Osaka 541-0045 (JP); YAMASAKI, Hiroyuki, Osaka-shi, Osaka 541-0045 (JP)
(74) Representative: Vossius & Partner Patentanwälte Rechtsanwälte mbB
(86) International application number: PCT/JP2024/033582
(87) International publication number: WO 2025/063261

(57) **Abstract**

It is an object of the present invention to provide novel polynucleotides that allow efficient expression of HIV Tat protein or antigenic fragment thereof and that have excellent ability to induce anti-Tat antibodies, as well as a pharmaceutical composition comprising the polynucleotides.

The present invention discloses novel polynucleotides encoding a polypeptide comprising (a) an HIV Tat protein or an antigenic fragment thereof, and (b) a signal peptide. The pharmaceutical composition comprising the polynucleotides can be used for the treatment and/or prevention of HIV-related diseases.

## Description

### Technical Field

The present invention relates to novel polynucleotides encoding a polypeptide comprising (a) a human immunodeficiency virus (HIV) trans-activator of transcription (Tat) protein or an antigenic fragment thereof, and (b) a signal peptide, and to pharmaceutical compositions comprising the polynucleotide.

### Background Art

Human immunodeficiency virus (HIV), a retrovirus, is known to cause acquired immunodeficiency syndrome (AIDS). HIV consists of only 14 genes, two of which are known as HIV-1 and HIV-2. HIV-1 and HIV-2 commonly encode structural proteins (Gag and Env), enzymes necessary for replication (Pol), and regulatory proteins (Tat and Rev) essential for virus growth.

Therapeutic agents for AIDS include nucleic acid-based reverse transcriptase inhibitors (tenofovir alafenamide, emtricitabine, abacavir, lamivudine, etc.), non-nucleic acid reverse transcription inhibitors (such as rilpivirine, ephevirenz, doravirine, etc.), protease inhibitors (such as darunavir, ritonavir, etc.), and integrase inhibitors (such as dolutegravir, raltegravir, bictegravir, elvitegravir) are mainstream. In recent years, a combination anti-retroviral therapy (cART) has been established to treat them in combination, and the life expectancy of people infected with HIV has been remarkably improved.

On the other hand, with the aging of HIV patients, HIV-related neurocognitive disorder (HAND), which was previously not focused on as a research target, has emerged. In Non Patent Documents 1 and 2, HAND refers to a disease associated with cognitive deficits, motor deficits and abnormal behavior, despite a successful treatment of HIV, and it is described that HAND is found in about 30% of HIV infected people. One of the causes of HAND is the toxicity of the Tat protein, which is essential for the transcription of HIV genes, to nerve cells and the long-term infection of HIV may alter the blood-brain barrier. It has been reported that Tat protein forms complexes with amyloid β peptide to enhance adhesion to nerve cells, and larger complexes adhere to the surface of the nerve. In addition, in people expressing the Tat protein in the brain, there is an inverse correlation between the amount of anti-Tat antibody and the severity of HAND, and higher anti-Tat titers have been shown to milder the symptoms of HAND. Thus, vaccines that induce anti-Tat antibodies are expected to be effective as therapeutic agents for HAND.

Non Patent Document 3 and Patent Document 6 describe Tat protein vaccines that induce anti-Tat antibodies and describe that at least three administrations are preferred for anti-Tat antibody induction. Therefore, a need remains for Tat vaccines that have better antibody inducing capacity with fewer administrations.

In recent years, mRNA vaccines have attracted attention in the field of vaccines.

Patent Documents 1, 2, and 5 describe mRNA encoding Tat protein, which is characterized by including a histone stem loop, but does not at all disclose specific sequence or biological data of the mRNA encoding the Tat protein.

Patent Document 3 describes a composition comprising mRNA encoding two or more pathogenic antigens, and describes a Tat antigen as an example of a pathogenic antigen. However, no specific sequence or biological data of the mRNA encoding the Tat antigen and other pathogenic antigens has been disclosed. Patent Document 4 describes a polynucleotide that encodes a secretory protein mutant obtained by adding a secretory signal sequence and a self-penetrating peptide sequence derived from the Tat protein (an amino acid sequence of 11 residues represented by SEQ ID NO: 9 in Patent Document 4) to a non-secretory protein that is normally present only intracellularly. Here, the Tat peptide is added in cooperation with the secretory signal sequence to allow the non-secretory protein to pass through the cell membrane and the blood-brain barrier.

### Prior Art Documents

### Patent Documents

Patent Document 1: WO 2013/120628
Patent Document 2: WO 2012/019780
Patent Document 3: WO 2022/212659
Patent Document 4: WO 2023/115194
Patent Document 5: WO 2013/120499
Patent Document 6: WO 2011/113618
Patent Document 7: WO 2017/081082
Patent Document 8: WO 2023/218420

### Non Patent Documents

Non Patent Document 1: Hategan et al., Nat Struct Mol Biol, 2017(24), 379-386
Non Patent Document 2: Omeragic et al., Fluids Barriers CNS (2020) 17: 42
Non Patent Document 3: Cafaro et al., Expert Opin Biol Ther. 2015; 15 Suppl 1: S13-29
Non Patent Document 4: Fabienne et al., EMBO, 2010(29), 1348-1362
Non Patent Document 5: Annunziata et al., J Cell Biochem, 2013(114), 510-513

### SUMMARY OF THE INVENTION

### TECHNICAL PROBLEMS TO BE SOLVED BY THE INVENTION

It is an object of the present invention to provide novel polynucleotides encoding HIV Tat proteins or antigenic fragments thereof that can be utilized as therapeutic and/or prophylactic agents for HIV-associated diseases (for example, HIV infection and HAND).

### MEANS FOR SOLVING THE PROBLEM

In the field of mRNA vaccines, as one method for enhancing the antigenicity of a protein encoded by mRNA, it is known to optimize the secretory signal peptide sequence at the terminal of the amino acid sequence inherently possessed by the protein and increase the amount of peptide transported to the endoplasmic reticulum, thereby enabling efficient antigen presentation.

The Tat protein does not have a secretory signal peptide sequence, and an arginine-rich domain is important for the extracellular secretion of the Tat protein. It is said that the Tat protein binds to phosphatidylinositol-4,5-bisphosphate (Phosphatidylinositol-4,5-bisphosphase, PI (4,5) P2) in the cell membrane and is inserted into the cell membrane to be secreted outside the cell without the endoplasmic reticulum (Non Patent Document 4). Therefore, even if the signal sequence is added to the mRNA encoding the Tat protein, the amount of Tat protein to be transported into the endoplasmic reticulum does not necessarily increase, and the antigenicity of the mRNA is not necessarily improved. Furthermore, there is no case where a secretory signal sequence is added to the mRNA that encodes the Tat protein. It is generally considered that artificially introducing a secretory signal peptide to a protein that does not have a secretory signal peptide may lead to a breakdown of functions such as folding of the protein, regulation of secretory pathway and quantity of secretion (Non Patent Document 5).

Patent Document 8 was published on November 16, 2023, which is after the priority date of the present application.

The inventors have made extensive research and found that novel polynucleotides encoding a polypeptide which comprises (a) an HIV Tat protein or an antigenic fragment thereof and (b) a signal peptide (hereinafter also referred to as "polynucleotides of the present invention"). As described in Examples below, the polynucleotide can efficiently express HIV Tat protein and has an excellent ability to induce anti-Tat antibodies. Further from the Examples below, it is suggested that pharmaceutical compositions comprising a polynucleotide of the present invention have a therapeutic and/or prophylactic effect of HIV-associated diseases (for example, HIV infection and HAND).

That is, the present invention relates to the following.
(1-1) A polynucleotide encoding a polypeptide comprising the following (a) and (b):
   (a) an HIV Tat protein or antigenic fragment thereof; and
   (b) a signal peptide.
(1-2) The polypeptide according to (1-1), wherein the signal peptide is a secretory signal peptide.
(1-3) The polynucleotide according to (1-2), wherein the secretory signal peptide is an N-terminal secretory signal peptide.
(2-1) The polynucleotide according to (1-2), wherein the secretory signal peptide is (i) or (ii):
   (i) a polypeptide consisting of an amino acid sequence set forth in any one of SEQ ID NOs: 161 to 167 and 183 to 187; or
   (ii) a polypeptide consisting of an amino acid sequence in which one or more amino acids are deleted, substituted, inserted, or added in an amino acid sequence set forth in any one of SEQ ID NOs: 161 to 167 and 183 to 187 and having secretory signal activity.
(2-2) The polynucleotide according to any one of (1-1) to (2-1), further comprising (c) a polynucleotide encoding a polypeptide comprising a functional sequence.
(2-3) The polynucleotide according to (2-2), wherein the functional sequence is (iii) or (iv):
   (iii) a polypeptide consisting of an amino acid sequence of SEQ ID NO: 168 or 169; or
   (iv) a polypeptide consisting of an amino acid sequence in which one or more amino acids in an amino acid sequence set forth in SEQ ID NO: 168 or 169 have been deleted, substituted, inserted or added.
(3) The polynucleotide according to any one of (1-1) to (2-3), wherein (a) is an HIV Tat protein.
(4) The polynucleotide according to any one of (1-1) to (3), wherein the HIV Tat protein is an HIV-1 Tat protein.
(5-1) The polynucleotide according to (4), wherein the HIV-1 Tat protein is a polypeptide consisting of an amino acid sequence that is identical to, or having 90% or greater identity with, the amino acid sequence set forth in any one of SEQ ID NOs: 131 to 148 and 182.
(5-2) The polynucleotide according to (5-1), wherein the HIV-1 Tat protein is a polypeptide consisting of an amino acid sequence set forth in SEQ ID NO: 133 or 134.
(6-1) The polynucleotide according to any one of (1-1) to (5-2), wherein the polypeptide comprising (a) and (b) is a polypeptide consisting of an amino acid sequence set forth in any one of SEQ ID NOs: 168 to 178 and 190 to 197.
(6-2) The polynucleotide according to (6-1), wherein the polypeptide comprising (a) and (b) is a polypeptide consisting of an amino acid sequence set forth in any one of SEQ ID NOs: 174 to 178.
(6-3) The polynucleotide according to (6-2), wherein the polypeptide comprising (a) and (b) is a polypeptide consisting of an amino acid sequence of SEQ ID NO: 174.
(7-1) The polynucleotide according to any one of (1-1) to (6-3), wherein the polynucleotide is an mRNA comprising an open reading frame encoding a polypeptide comprising the following (a) and (b) (hereinafter also referred to as "mRNA of the present invention", the "mRNA of the present invention" is comprised within "polynucleotides of the present invention"):
   (a) an HIV Tat protein or antigenic fragment thereof; and
   (b) a secretory signal peptide.
(7-2) The polynucleotide according to any one of (1-1) to (7-1), wherein the polynucleotide is an mRNA comprising a 5' Cap structure, a 5' UTR, an open reading frame encoding a polypeptide comprising the following (a) and (b), a 3' UTR, and a Poly(A):
   (a) an HIV Tat protein or antigenic fragment thereof; and
   (b) a secretory signal peptide.
(8-1) The polynucleotide according to (7-1) or (7-2), further comprising: a modified base selected from 5-methyluridine, pseudouridine, N1-methylpseudouridine, 5-methoxyuridine, 2-thiouridine, 6-methyladenosine, 2-aminoadenosine, inosine, 5-methylcytidine, N1-ethylpseudouridine, 4-thiouridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxypseudouridine, 4-thio-1-methyl-pseudouridine, 4-thio-pseudouridine, 5-aza-uridine, dihydropseudouridine, 2'-O-methyluridine, or a combination thereof.
(8-2) The polynucleotide according to (8-1), wherein the modified base is selected from 5-methyluridine, N1-methylpseudouridine, 5-methylcytidine, or a combination thereof.
(9) The polynucleotide according to (7-1) to (8-2), further comprising a 5' Cap structure that is Cap1 or Cap2.
(10) The polynucleotide according to (9), wherein the 5' Cap structure is Cap1.
(11-1) The polynucleotide according to any one of (7-1) to (10), further comprising Poly(A) of 60 to 160 mers.
(11-2) The polynucleotide according to (11-1), wherein Poly(A) is 80 mers.
(12-1) The polynucleotide according to any one of (7-1) to (11-2), further comprising a 5' UTR that is a polynucleotide consisting of a nucleotide sequence identical to, or having 90% or greater identity with, a nucleotide sequence set forth in any one of SEQ ID NOs: 1 to 63 and 211 to 218.
(12-2) The polynucleotide according to (12-1), wherein the 5' UTR is a polynucleotide consisting of a nucleotide sequence identical to, or having 90% or greater identity with, a nucleotide sequence set forth in any one of SEQ ID NOs: 1 to 9.
(12-3) The polynucleotide according to (12-2), wherein the 5' UTR is a polynucleotide consisting of a nucleotide sequence identical to, or having 90% or greater identity with, a nucleotide sequence set forth in any one of SEQ ID NOs: 1 to 6.
(13-1) The polynucleotide according to any one of (7-1) to (12-3), further comprising a 3' UTR that is a polynucleotide consisting of a nucleotide sequence identical to, or having 90% or greater identity with, a nucleotide sequence set forth in any one of SEQ ID NOs: 64 to 112.
(13-2) The polynucleotide according to (13-1), wherein the 3' UTR is a polynucleotide consisting of a nucleotide sequence identical to, or having 90% or greater identity with, the nucleotide sequence set forth in any one of SEQ ID NOs: 64 to 69.
(14-1) The polynucleotide according to any one of (7-1) to (13-2), wherein the open reading frame consists of a polynucleotide having a nucleotide sequence set forth in any one of SEQ ID NOs: 114 to 119, 121 to 126, and 198 to 210.
(14-2) The polynucleotide according to (14-1), wherein the open reading frame consists of a polynucleotide having a nucleotide sequence set forth in any one of SEQ ID NOs: 114 to 119, 121 to 125, and 198 to 203.
(15-1) The polynucleotide according to any one of (7-1) to (14-2), wherein the polynucleotide is an mRNA defined by any one of SNG-299, 322 to 325, 327, 328, 331 to 336, 338 to 341, and 345.
   (That is, the polynucleotides described in any of Tables 5, 6-1, and 6-2 of Example 7 below.)
(15-2) The polynucleotide according to (15-1), wherein the polynucleotide is an mRNA defined by any one of SNG-299, 322 to 325, 327, and 328.
   (That is, polynucleotides as described in Table 6-2 of Example 7 below.)
(16) A pharmaceutical composition comprising the polynucleotide according to any one of (1-1) to (15-2) (hereinafter also referred to as "pharmaceutical composition of the present invention").
(17-1) The pharmaceutical composition according to (16), wherein the pharmaceutical composition is a therapeutic and/or prophylactic agent for an HIV-associated disease.
(17-2) The pharmaceutical composition according to (17-1), wherein the HIV-associated disease is an HIV infection or HAND.
(18) A method of treating and/or preventing an HIV-associated disease, the method comprising administering the polynucleotide according to any one of (1-1) to (15-2).
(19) The polynucleotide according to any one of (1-1) to (15-2), for use in the manufacture of a therapeutic agent and/or a prophylactic agent for an HIV-related disease.
(20) The polynucleotide according to any one of (1-1) to (15-2) for use in treatment and/or prevention of an HIV-associated disease.
(21) Use of the polynucleotide according to any one of (1-1) to (15-2) for treatment and/or prevention of an HIV-associated disease.

### EFFECT OF THE INVENTION

Since the polynucleotide of the present invention efficiently expresses HIV Tat protein and has excellent ability to induce anti-Tat antibodies, the pharmaceutical composition comprising the polynucleotide is highly useful as a pharmaceutical for use in treatment and/or prevention of HIV-associated diseases (for example, HIV infection and HAND).

### MODE FOR CARRYING OUT THE INVENTION

Hereinafter, the meaning of each term used in the present specification will be described. Unless particularly stated otherwise, each term is used in the same sense, either alone or in combination with other terms.

The term "consist of" means having only the constituent elements.

The term "comprise" means that elements are not limited to the constituent elements, and elements that are not described are not excluded.

The present invention will be described below with reference to embodiments. Throughout this specification, it should be understood that the representation of the singular also includes the concept of the plural unless stated otherwise. Thus, unless stated otherwise, singular articles (for example, "a", "an", "the", and the like in English) can be understood to include the concepts of the plural forms.

Unless otherwise defined, it should be understood that the terms used in the present specification are used in a meaning normally used in the art. Accordingly, unless otherwise defined, all terminologies and scientific and technical terms used in the present specification have the same meanings as commonly understood by those having ordinary skill in the art to which the present invention belongs. In the event of any inconsistency, the present specification (including the definitions) shall prevail.

The term "polypeptide" means a polymer of amino-acid residues (natural or non-natural) linked by peptide bonds, and refers to a protein having any size, structure, or function. When the encoded polypeptide is smaller than about 50 amino acids, it is called a peptide, and when the polypeptide is a peptide, it is at least about 2, 3, 4, or at least 5 amino-acid residues in length. Thus, the polypeptide may comprise a gene product, a naturally occurring polypeptide, a synthetic polypeptide, homologues, orthologs, paralogs, the above-mentioned fragments and other equivalents, variants and analogs. The polypeptide may be a single molecule or multimolecular complex such as a dimer, a trimer or a tetramer.

An "HIV Tat protein" is a protein present in both HIV-1 and HIV-2 and plays an important role in the regulation and replication of the respective gene expressions. An HIV Tat protein comprises a proline-rich domain, a cysteine-rich domain, a core domain, an arginine-rich domain and a glutamine-rich domain in exon 1, and comprises an RGD motif in exon 2. Although it differs among isolates, it is known that the HIV-1 Tat protein consists of 99 to 106 residues and includes M, O, and N groups and the M group mainly includes A to D subtypes, the CRF01 AE subtypes, the F to H subtypes, the J and K subtypes, and the like. On the other hand, although it also differs among isolates, it is known that an HIV-2 Tat protein is a protein consisting of about 130 residues and mainly includes A to G subtypes. The HIV-1 Tat and HIV-2 Tat genes both consist of two exons that encode the protein. The amino acid sequences of HIV-1 Tat proteins include SEQ ID NOs: 129 to 130 (A subtype), SEQ ID NOs: 131 to 148 (B subtype), SEQ ID NOs: 149 to 153 (C subtype), SEQ ID NOs: 154 to 156 (D subtype), SEQ ID NOs: 157 to 158 (subtype E), and the like. The amino acid sequence of the HIV-2 Tat protein includes SEQ ID NO: 159 and the like.

The HIV Tat protein is preferably HIV-1 Tat B subtype, more preferably HIV-1 Tat Oyi or BH10. The amino acid sequence of the HIV Tat protein is preferably the one set forth in SEQ ID NOs: 131 to 148, and more preferably the amino acid sequence set forth in SEQ ID NO: 133 (HIV-1 Tat Oyi) or SEQ ID NO: 134 (HIV-1 Tat BH10).

In one embodiment, an HIV Tat protein encoded by the polynucleotide of the present invention may be a chimeric protein of HIV Tat, an HIV Tat protein with a mutation in an arginine-rich domain, an HIV Tat protein with a mutation in an RGD motif, a partial deletion mutant of HIV Tat protein, or a conjugate of bivalent or higher HIV Tat protein.

The term "HIV Tat chimeric protein" means a protein comprising or consisting of fragments derived from HIV Tat protein of two or more different subtypes, or a chimeric protein of an HIV Tat protein and an SIV Tat protein. The HIV Tat chimeric protein can be, for example, a chimeric protein comprising/consisting of a protein in which a protein derived from exon 1 of the HIV Tat is linked to the C-terminus of a protein derived from exon 2 of the HIV Tat. The HIV Tat chimeric protein is preferably a chimeric protein derived from HIV-1 Tat, which comprises exon 2 of a protein derived from HIV-1 Tat C subtype. The HIV Tat chimeric protein is more preferably a chimeric protein comprising a protein derived from exon 1 of the HIV-1 Tat B subtype and a protein derived from exon 2 of the HIV-1 Tat C subtype.

In one embodiment, the HIV Tat chimeric protein can be a chimeric protein of a protein derived from the Tat B subtype of HIV-1 and a SIV Tat protein.

In one embodiment, the HIV Tat chimeric protein can be a chimeric protein of a protein derived from exon 1 of HIV Tat and exon 2 of HIV Tat or SIV Tat.

The antigenic fragment of the chimeric protein may have about 1 to 10 amino acids inserted between the protein derived from the N-terminus, C-terminus, or exon 1 and the protein derived from exon 2, to such an extent as not to lose the antigenicity of the HIV Tat protein.

The term "HIV Tat protein with a mutation in an arginine-rich domain" means an HIV Tat protein obtained by introducing a mutation into the arginine-rich domain of the wild-type HIV Tat protein. The arginine-rich domain of HIV Tat protein has been reported to be cleaved by the cell-derived protease Furin. The HIV Tat protein with a mutation in an arginine-rich domain can be expected to increase its stability as an antigen by eliminating cleavage by Furin.

The term "HIV Tat protein with a mutation in an RGD motif" means an HIV Tat protein obtained by introducing a mutation into the RGD motif present in exon 2 of the wild-type HIV Tat protein. It has been reported that HIV Tat protein induces cell death in nervous system cells and immune system cells through the RGD motif of exon 2. The HIV Tat protein with a specific mutation in the RGD motif is expected to lose or attenuate the inducing activity of cell death in nervous system cells and immune system cells, and can induce immunity having the same neutralizing activity as that of HIV Tat protein without mutations. For example, the HIV Tat protein with a mutation in an RGD motif can be a protein consisting of an amino acid of SEQ ID NO: 182 or the like.

The term "partial deletion mutant of HIV Tat protein" means an antigen in which the partial functional domain of the HIV Tat protein has been deleted. The antigen with a partial functional domain deleted can be an antigen obtained by deleting the region comprising a cysteine-rich domain, arginine-rich domain, or RGD motif from the wild-type amino acid sequence of HIV Tat protein. It is known that the cysteine-rich domain is involved in the transcriptional activation of HIV Tat protein, the arginine-rich domain is involved in extracellular transport, and the region comprising an RGD motif (exon 2 region) is involved in cell death induction. Therefore, the partial deletion mutant of the HIV Tat protein with a region comprising a cysteine-rich domain, arginine-rich domain, or an RGD motif deleted lacks the above-described function of the HIV Tat protein, and is expected to only act as an antigen.

The term "conjugate of bivalent or higher HIV Tat protein" means a multimer in which bivalent or higher HIV Tat proteins are serially linked in a tandem manner, which may be via a linker of several amino acids in the connection. Preferably, it is full-length HIV Tat protein of tandemly linked 2- to 10-valent units, more preferably tandemly linked 2- to 6-valent units, and still more preferably a 3- or 5-valent unit. Here, the HIV Tat protein may be not only a wild-type, but also a protein that has a mutation in the arginine-rich domain and/or an RGD motif. Even if the arginine-rich domain undergoes cleavage by Furin during the extracellular transport process of Tat protein, it is expected that conjugate of bivalent or higher HIV Tat protein will be expressed in a form close to the full-length HIV Tat protein, thereby maintaining antigenicity. Furthermore, improvement of antigenicity is expected.

The term "SIV Tat protein" is a protein that is present in the simian immunodeficiency virus and has similar functions as the HIV Tat protein. The amino acid sequence of the SIV Tat protein can be SEQ ID NO: 160 or the like.

The "antigen" means a substance which can be recognized by the immune system, preferably by an adaptive immune response. As part of the adaptive immune response, antigen-specific immune responses are induced by induction of antibodies and antigen-specific T cells. The antigen can be a peptide or protein that can be presented to T cells by MHC, a translation product of polynucleotide encoding such peptide or protein. Examples of the antigen include fragments of peptides and proteins containing the epitope, mutants and derivatives of the fragments, and the like.

The term "antigenic fragment of HIV Tat protein" means a fragment of an HIV Tat protein that includes part of the HIV Tat protein and, like the full-length HIV Tat protein, is recognized by immune cells such as T cells and B cells, and can induce an immune response such as antibody production against the HIV Tat protein. The antigenic fragment of HIV Tat protein can be a fragment of the HIV Tat protein having 1 to 20, 1 to 15, 1 to 10, 1 to 8, 1 to 6, 1 to 4, 1 to 2 residues deleted from the N-terminus, central, and/or C-terminus amino acids.

The "polynucleotide" means a polymer of nucleotide of any length, comprising a ribonucleotide, a deoxyribonucleotide, an analog thereof, or a mixture thereof. The polynucleotide includes a triple-stranded, double-stranded, and single-stranded deoxyribonucleic acids (DNA), and triple-stranded, double-stranded, and single-stranded ribonucleic acids (RNA). The polynucleotide further includes polynucleotides and circular polynucleotides in forms modified by alkylation and/or capping as well as in an unmodified form. Examples of the polynucleotide include polydexyribonucleotides containing 2-deoxy-D-ribose; polyribonucleotides such as tRNA, rRNA, hRNA, siRNA, and mRNA containing D-ribose; other types of polynucleotides that are N-glycosides or C-glycosides of purine or pyrimidine bases; and other polymers containing a non-nucleotide backbone. The polymer can be polyamides such as the peptide nucleic acid PNA, polymorpholino polymers, other synthetic sequence-specific nucleic acid polymers, or the like. The mRNA includes IVT mRNA, self-replicating RNA, and replicon RNA. The self-replicating RNA and replicon RNA include ssRNA viruses, such as a positive-stranded ssRNA virus.

The polynucleotide of the present invention is preferably a ribonucleotide or deoxyribonucleotide, and more preferably mRNA. Here, the polynucleotide of the present invention all refers to non-naturally occurring polynucleotide, and mRNA of the present invention all refers to non-naturally occurring mRNA.

The term "non-naturally occurring mRNA" means an mRNA that differs by at least one nucleotide from a wild-type sequence present in nature. The mRNA can be produced by conventional genetic modification techniques. In one embodiment, the non-naturally occurring mRNA comprises at least one modified base. In one embodiment, all nucleobases are replaced with modified bases. In one embodiment, the polynucleotide (for example, synthetic RNA or synthetic DNA) may consist solely of naturally occurring nucleobases, that is, in the case of synthetic DNA, bases A, C, G, and T, and in the case of synthetic RNA, bases A, C, G, and U.

The term "functional sequence" means a polypeptide of 3 to 60 amino acids which is located at the N-terminus, internally, or C-terminus of a protein or peptide fragment thereof, and which may define the secretory pathway and localization of the protein or peptide fragment thereof. The functional sequence is synonymous with the heterologous peptide. Depending on the position of a functional sequence within a protein or a peptide fragment thereof, the functional sequence is classified as an N-terminal functional sequence, an internal functional sequence, or a C-terminal functional sequence.

The functional sequences can be a signal peptide, a linker peptide, an immuno-activating helper epitope, a TLR-activating peptide, a multimerization domain peptide, such as that set forth in SEQ ID NO: 189, or the like.

The signal peptide can be a secretory signal peptide, a nuclear translocation signal peptide, a nuclear localization signal peptide, a ubiquitination signal peptide, an endoplasmic reticulum-targeting signal peptide such as that set forth in SEQ ID NO: 188, a plasma membrane-targeting signal peptide, a Golgi apparatus-targeting signal peptide, an endosome-targeting signal peptide, a cytoskeleton-targeting signal peptide, a cytoplasm-targeting signal peptide, or the like.

The "secretory signal peptide (SSPs)" means a short peptide as short as about 15 to 30 amino acids, which is located at the N-terminus, an internal region, or C-terminus of a protein or peptide fragment thereof and promotes transport of the protein or peptide fragment into the endoplasmic reticulum, and has a secretory function. The secretory signal peptide may be operably linked (fused) to the protein or a peptide fragment thereof, or may be linked to a polypeptide which has a methionine removed from the N-terminus of the protein or peptide fragment thereof.

The amino acid sequence of a secretory signal peptide encoded by the polynucleotide of the present invention can be a polypeptide consisting of an amino acid sequence set forth in any one of SEQ ID NOs: 1 to 1115 and 1728 in Patent Document 7 and SEQ ID NOs: 161 to 167 and 183 to 187 in the present specification, a polypeptide consisting of an amino acid sequence in which one or more amino acids are deleted, substituted, inserted, or added in the amino acid sequence and having secretory signal activity, or the like. The addition means that one or more amino acids are linked to the N- or C-terminus of the amino acid sequence. The term "one or more" refers to 1 to 10, preferably 1 to 5.

The secretory signal activity is preferably an N-terminal secretory signal peptide consisting of an amino acid sequence deleted, substituted, inserted, or added with one or more amino acids in the amino acid sequences set forth in any one of SEQ ID NOs: 161 to 167 in the present specification and having secretory signal activity. The secretory signal activity is more preferably, IgE (SEQ ID NO: 161), Igk (SEQ ID NO: 162), JEV (SEQ ID NO: 163), SARS (SEQ ID NO: 164), IgG (SEQ ID NO: 167), tPA (SEQ ID NO: 165), albumin (SEQ ID NO: 166), Cystatin S (SEQ ID NO: 183), tPA (SEQ ID NO: 165), Mod-tPA (SEQ ID NO: 184), IL-6 (SEQ ID NO: 185), CTLA4 (SEQ ID NO: 186), or HSV/gDsec (SEQ ID NO: 187). The secretory signal activity is more preferably IgE, Igk, JEV, SARS, tPA, or IgG. The secretory signal activity is still more preferably IgE, JEV, or SARS. The secretory signal activity is particularly preferably IgE.

In one embodiment, the polynucleotide of the present invention may encode an HIV Tat protein or an antigenic fragment thereof, in which a secretory signal peptide is linked at the N-terminus and a functional sequence is linked at the C-terminus.

The term "5' UTR" is synonymous with 5'-untranslated region and means a region of mRNA located between the 5' Cap structure and the start codon on the mRNA being translated by the ribosome. Typically, the 5' UTR means a region from a nucleotide adjacent to the 3' end of a 5' Cap structure up to a nucleotide adjacent to the 5' end of the start codon on the open reading frame (ORF). The 5' UTR may include a regulatory element of gene expression, a protein binding region, and the like. The regulatory element can be a ribosomal binding site, a miRNA binding site, a 5'-terminal oligopyrimidine tract, or the like.

The 5' UTR of mRNA of the present invention can be a polynucleotide consisting of a nucleotide sequence identical to, or having 80%, 85%, 90%, or 95% or greater identity with, a nucleotide sequence set forth in any one of SEQ ID NOs: 1 to 63 and 211 to 218. The 5' UTR of mRNA of the present invention is preferably a polynucleotide comprising a nucleotide sequence identical to, or having 90% or greater identity with, a nucleotide sequence set forth in any one of SEQ ID NOs: 1 to 9, more preferably a polynucleotide comprising a nucleotide sequence identical to, or having 90% or greater identity with, a nucleotide sequence set forth in any one of SEQ ID NOs: 1 to 6. The 5' UTR of mRNA of the present invention is particularly preferably a polynucleotide consisting of the same nucleotide sequence as that set forth in any one of SEQ ID NOs: 1 to 3.

The term "3' UTR" is synonymous with 3'-untranslated region and means a region of mRNA downstream (3' side) of the stop codon on the mRNA being translated by the ribosome. The 3' UTR may include a regulatory element of gene expression, a protein binding region, a PolyA addition signal sequences, and the like. The regulatory element can be a ribosomal binding site, a miRNA binding site, or the like.

The 3' UTR of mRNA of the present invention can be, for example, a polynucleotide consisting of a nucleotide sequence identical to, or having 80%, 85%, 90%, or 95% or greater identity with a nucleotide sequence set forth in any one of SEQ ID NOs: 64 to 112. The 3' UTR of mRNA of the present invention is preferably a polynucleotide comprising a nucleotide sequence identical to, or having 90% or greater identity with a nucleotide sequence set forth in any one of SEQ ID NOs: 64 to 69, and more preferably a polynucleotide comprising a nucleotide sequence identical to, or having 90% or greater identity with a nucleotide sequence set forth in SEQ ID NOs: 64 to 66. The 3' UTR of mRNA of the present invention is particularly preferably a polynucleotide consisting of the same nucleotide sequence as those set forth in SEQ ID NOs: 64 to 66.

The 3' UTR of mRNA of the present invention may include a short nucleotide sequence such as a restriction enzyme cleavage sequence (GCUAGC) derived from a cloning site such as NheI at the 3' terminus. Each 3' UTR represented in SEQ ID NOs: 64 to 66 is a polynucleotide obtained by adding a NheI cleavage sequence to each 3' UTR represented in SEQ ID NOs: 67 to 69.

The term "identity" means the percentage of nucleotides or amino acids that match the same nucleotides or amino acids of the reference sequence at that sequence position. When two or more sequences have nucleotides or amino acids of the same length and in the same order, the sequences are described as being identical. When assessing identity, the sequences to be compared are considered to have the same length, that is, the length of the longest sequence among the sequences being compared. This means that a first sequence consisting of 9 nucleotides or amino acids is 90% identical to a second sequence consisting of 10 nucleotides or amino acids that includes the first sequence.

The identity of an amino acid sequence or a nucleotide sequence can be determined using software such as the BLAST algorithm by Karlin and Altschul (Proc. Natl. Acad. Sci. USA (1993) 90: 5873-7) or ClustalW2 provided by the European Molecular Biology Laboratory-European Bioinformatics Institute (EMBL-EBI).

The term "Poly(A)", also referred to as the PolyA tail or 3'-PolyA tail, means a sequence of about 10 to 500 mers of adenosine nucleotide that is directly downstream (i.e., 3' side) from the 3' UTR of the mRNA.

In one embodiment, the mRNA of the present invention may have the Amino Terminal Enhancer of Split (AES) sequence, mitochondrial-encoded 12S ribosomal RNA sequence, A30LA70 sequence, etc. inserted between the ORF and Poly(A).

In one embodiment, the mRNA of the present invention may have a mask structure made of a nucleotide other than adenyl (A) residue, INVERTED-dT, 2'-modified RNA, or the like inserted on the 3'-terminus side of Poly(A). The phosphodiester moiety of the Poly(A) tail may have phosphorothioate, phosphorodithioate, phosphoroselenate, or phosphorodiselenate linkage for the purpose of improving mRNA stability.

In one embodiment, the mRNA of the present invention may include a Poly(A) and/or histone stem-loop at the 3'-terminus side, and may be present in combination with other elements such as a purine-rich region (a histone downstream element, or HDE) .

The Poly(A) of mRNA of the present invention is an adenosine nucleotide of preferably 20 to 250 mers, more preferably 60 to 160 mers, and particularly preferably 75 to 85 mers, or 80 mers.

The term "open reading frame (ORF)" means a nucleotide sequence from the start codon to the stop codon. The stop codon needs to only include at least one stop codon, and may include any number of UAA, UAG, or UGA.

The ORF may have all convertible codons converted, and may be optimized using an optimization algorithm so that the mRNA forms a stable secondary structure. Codon optimization tools, algorithms, and services are known in the art and are described in services of GeneArt (Life Technologies), DNA 2.0 (Menlo Park CA), ViennaRNA Package 2.0 (University of Vienna), LinearDesign (Baidu Inc.), and the like.

The nucleotide sequence of the ORF of the polynucleotide of the present invention can be nucleotide sequences in which the G/C content is increased or codons are optimized without altering the amino acid sequence from a wild-type sequence, thereby increasing the expression level of the protein encoded by the ORF. In some embodiments of the polynucleotide of the present invention, the G/C content of an ORF is increased, for example, 5% or more, 10% or more, 15% or more, 20% or more relative to the G/C content of the ORF of a wild-type Tat polynucleotide.

The ORF of the polynucleotide of the present invention may encode polypeptides that include HIV-1 Tat protein and a secretory signal peptide. ORF can be mRNA encoding a polypeptide in which a secretory signal peptide is linked to an HIV-1 Tat protein in Example 3, for example.

The ORF sequence in the ORF encoding a protein comprising an HIV-1 Tat protein and a secretory signal peptide can be a nucleotide sequence set forth in SEQ ID NOs: 114 to 119, 121 to 126, 198 to 210, and the like, described in Example 3. The ORF sequence is preferably SEQ ID NOs: 121 to 126 and 202 to 210. The ORF sequence is more preferably the nucleotide sequence set forth in any one of SEQ ID NOs: 121 and 198 to 203.

The 5' Cap means naturally occurring 5' Cap and modified 5' Cap. The naturally occurring 5' Cap means a ribose guanosine residue (m7G) where the 7' position of guanine bases present in all eukaryotes is methylated. The m7G is linked with a 5'-terminal nucleotide of mRNA (hereinafter referred to as ppp) via a 5'-5'-triphosphate bond. The 5' Cap protects mRNA from degradation by exonucleases, facilitates the delivery of mRNA from nucleus to cytoplasm, and contributes to efficient translation of the protein encoded by the mRNA by playing an important role in assembly of the translation initiation complex. A modified 5' Cap means a 5' Cap that has been subjected to some chemical modification to a naturally occurring 5' Cap and has a function equivalent to or greater than that of the naturally occurring 5' Cap in terms of protein translation.

The modified 5' Cap can be modified guanosine (hereinafter referred to as [G]), or any of structures described in WO 2017/066793, WO 2023/007019, CN 116987137, and WO 2023/246860. The nucleoside in [G] can be a structure in which guanosine (m7G-3'OMe) in which the 7-position of guanine and the 3'-position of ribose are O-methylated or guanosine (m7G-2'OMe) in which the 7-position of guanine and the 2'-position of ribose are O-methylated are added, other guanine derivatives, a sugar modification, or the like. Examples of the guanine derivative include hypoxanthine, N1-methylguanine, 7-deaza-guanine, and 8-oxo-guanine. The sugar modification can be a ribose modified at the 2' and/or 3' positions, such as 2'-fluororibose, 2'-aminoribose, and 2'-azidoribose, bridged nucleic acids (BNA) having a bridging structure, or a modified riboses described in WO 2014/081507.

The m7G which is naturally occurring 5' Cap, and m7G-3' OMe and m7G-2' OMe, which are modified 5' Cap, are specifically represented by the following structures:

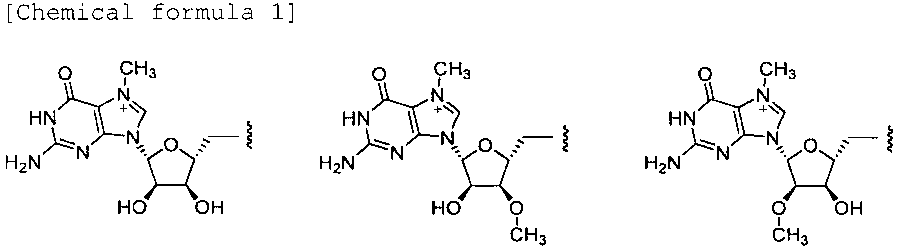

The term "5' Cap structure" as used herein means 5'-[G]pppN₁N₂N₃ (N₁, N₂, N₃ are any nucleoside) present at 5' terminus of mRNA.

The 5' Cap structure can be CapO, Cap1 (a structure in which the 2' position of N₁ is O-methylated, represented as 5'-[m7G]-ppp-N₁mN₂N₃), Cap2 (the 2' position of N₁ and N₂ is O-methylated, represented as 5'-[m7G]-ppp-N₁mN₂mN₃), Cap3 (a structure in which the 2' position of N₁, N₂, and N₃ is O-methylated, represented as 5'-[m7G]-ppp-N₁mN₂mN₃m) or the like. Specifically, Cap1 is represented by the following structure.

A Base in the chemical formula refers to any base. For example, the Cap1 may be GGG, AGG, AUA, or the like depending on a difference in the capping method. It has been confirmed that the expression levels in HEK293 cells and mice of mRNA with the 5' Cap structure expressed by 5'-[m7G]-ppp-GmGG, 5'-[m7G]-ppp-AmGG, or 5'-[m7G]-ppp-AmUA are equivalent.

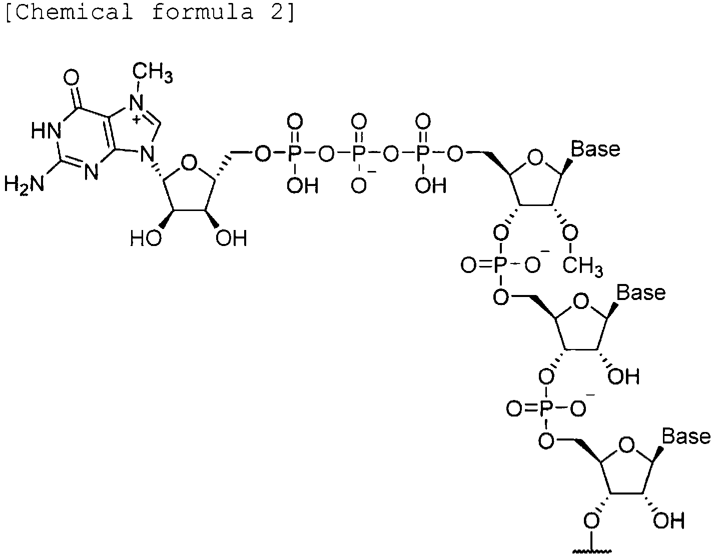

Cap2 is represented by the following structure. A Base in the chemical formula refers to any base.

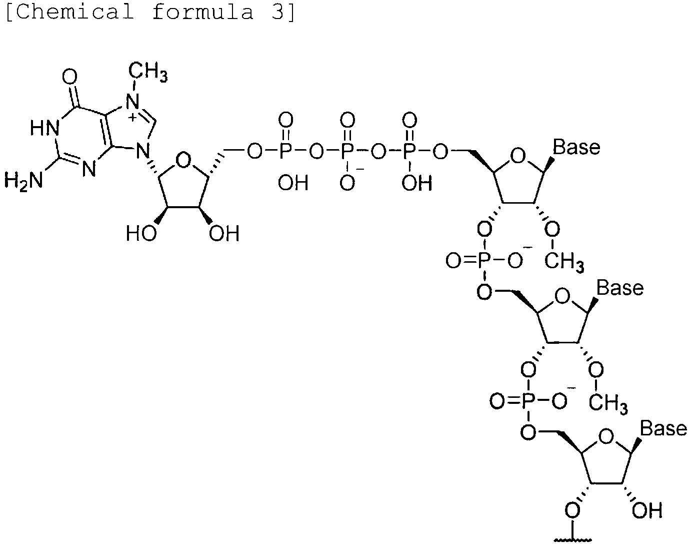

Cap3 is represented by the following structure. A Base in the chemical formula refers to any base.

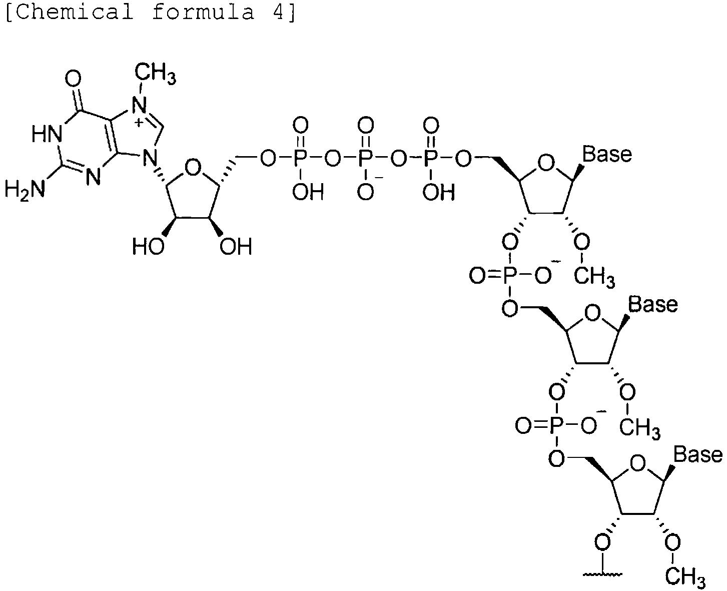

As the 5'-5'-triphosphate bond (ppp) that links the 5' Cap to the 5'-terminal nucleotide of mRNA, any of modified phosphate groups such as a phosphorothioate, phosphoroselenate, boranophosphate, boranophosphate ester, modified phosphate groups such as phosphonic hydrogen, phosphoramidate, alkyl or aryl phosphonates, and phosphotriesters can be employed.

The 5' Cap structure of mRNA of the present invention is preferably Cap1 or Cap2, particularly preferably Cap1.

The 5' Cap structure of mRNA in one embodiment of the present invention may have N₁N₂N₃ in 5'-[G]pppN₁N₂N₃ as GGG, AGG, AGA, AUG, GAA, or AUA, depending on the reagent used for capping. Thus, the nucleotide sequence of mRNA in an embodiment of the present invention begins with 5'-GGGG, 5'-GAGG, 5'-GAGA, 5'-GAUG, 5'-GGAA, or 5'-GAUA, followed by a 5' UTR sequence.

The mRNA of the present invention may have an internal ribosome entry site (IRES; internal ribosome entry site) instead of having a 5' Cap structure. Preferred IRES can be any of those derived from picornavirus, pestivirus, poliovirus, encephalomyocarditis virus, foot-and-mouth disease virus, hepatitis C virus, classical swine fever virus, murine leukemia virus, simian immunodeficiency virus, and cricket paralysis virus.

The polynucleotide of the present invention may include at least one chemical modification. As the chemical modification, any modification of polynucleotides known in the art may be used for the polynucleotides of the present invention, and examples thereof include modified phosphate groups, modified bases, and sugar modifications.

The modified phosphate group can be phosphorothioate, phosphoroselenate, boranophosphate, boranophosphate ester, hydrogen phosphonate, phosphoramidate, alkyl or aryl phosphonate, phosphotriester, or the like. The phosphorodithioate has both non-bound oxygen atoms replaced by sulfur. The phosphate linker may also be modified by replacing the bound oxygen with nitrogen (bridged phosphoramidate), sulfur (bridged phosphorothioate), and carbon (bridged methylene-phosphonate).

Examples of the modified base include 5-methyluridine, pseudouridine (Ψ), N1-methylpseudouridine (m1Ψ), 5-methoxyuridine, 2-thiouridine, 6-methyladenosine, 2-aminoadenosine, inosine, 5-methylcytidine, N1-ethylpseudouridine, 4'-thiouridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, and dihydropseudouridine. Further, modified bases described in WO 2001/79502, WO 2002/98443, WO 2009/127230, US 20100249219, WO 2012/019168, WO 2012/138453, WO 2013/143698, WO 2015/089511, US 20150056253, WO 2015/077123, WO 2016/209966, WO 2017/070613, US 20180126003, WO 2020/243002, WO 2021/198258, WO 2021/251453, WO 2022/233880, WO 2023/006999, and the like may also be used. The polynucleotide of the present invention may include multiple types of modified bases.

The modified base of mRNA of the present invention is a modified base selected from 5-methyluridine, pseudouridine, N1-methylpseudouridine (m1Ψ), 5-methoxyuridine, 2-thio-uridine, 6-methyladenosine, 2-aminoadenosine, inosine, 5-methylcytidine, N1-ethylpseudouridine, 4-thiouridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxypseudouridine, 4-thio-1-methyl-pseudouridine, 4-thio-pseudouridine, 5-aza-uridine, dihydropseudouridine, 2'-O-methyluridine, or a combination thereof. The modified base is more preferably 5-methyluridine, 5-methylcytidine, N1-methylpseudouridine, or a combination thereof. The modified base is particularly preferably N1-methylpseudouridine.

The sugar modification can be a nucleoside having a substituent at the 2' position of the sugar and/or a nucleoside having a bridging structure between 4' and 2' positions of the sugar.

The substituent on the 2'-hydroxy group of a sugar can be oxy or deoxy substituent. Examples of "oxy" modification include an alkoxy group or aryloxy (-OR such as R=H, alkyl, cycloalkyl, aryl, aralkyl, heteroaryl or sugar), polyethylene glycol (PEG), and aminoalkoxy. The "deoxy" modification can be hydrogen, amino, halogen (for example, fluorine and the like), or the like. The amino can be any of alkylamino, dialkylamino, heterocyclyl, arylamino, diarylamino, heteroarylamino, diheteroarylamino, amino acids, and the like. The amino may be bound to the sugar via a linker, and the linker includes at least one of atoms C, N, and O.

The bridging structure can be bridged nucleic acid (BNA) or the like. The BNA can be amido-bridged nucleic acid (AmNA, see WO 2011/052436), triazole-bridged nucleic acid (TrNA, see WO 2014/126229), 4'-(CH₂)ₙ-O-2' (n is an integer from 1 to 4), 4'-(CH₂)ₘ-C(=O)-NR₁-2' (m is an integer from 0 to 4, Rₗ is hydrogen atom or alkyl). Specific examples and the preparation method are described in WO 98/39352, WO 2003/068795, WO 2005/021570, WO 2011/052436, and WO 2013/052523.

The modification and modification method of nucleotides known in the art are also disclosed, for example, in the following patent documents:
WO 98/39352, WO 99/014226, WO 2000/056748, WO 2003/068795, WO 2004/016749, WO 2005/021570, WO 2005/083124, WO 2007/143315, WO 2009/071680, WO 2011/052436, WO 2014/112463, WO 2014/126229, and the like.

The polynucleotide of the present invention can be synthesized by conventional methods. For example, it may be synthesized by any method that may be synthesized, but not limited to, by chemical synthesis, enzymatic synthesis, generally referred to as in vitro transcription (IVT), or enzymatic or chemical cleavage of longer precursors. The synthesis is disclosed in, for example, the present specification, Gait, M.J. (ed.) Oligonucleotide synthesis: a practical approach, Oxford [Oxfordshire], Washington, DC: IRL Press, 1984, Herdewijn, P. (ed.) Oligonucleotide synthesis: methods and applications, Methods in Molecular Biology, v.288 (Clifton, N.J.) Totowa, N.J.: Humana Press, 2005), and the like.

The mRNA of the present invention can be synthesized by conventional methods. For example, the mRNA of the present invention can be synthesized by performing IVT on the basis of a template DNA that encodes the mRNA sequence of the present invention. When mRNA is synthesized by IVT, a template DNA is required. The template DNA may be a plasmid linearized by a restriction enzyme or the like, or a DNA fragment produced by a PCR method.

The template DNA of the mRNA of the present invention may comprise, from the 5'-terminus side, a promoter sequence of RNA polymerase (for example, T7 promoter sequence, SP6 promoter sequence, T3 promoter sequence, and the like), a 5'-UTR, an ORF region, and the 3'-UTR. The template DNA may also include a moiety corresponding to Poly(A), and by the DNA template including a moiety corresponding to Poly(A), the moiety corresponding to Poly(A) is synthesized through RNA synthesis reaction with RNA polymerase. As another method for adding Poly(A) to mRNA, there is a method in which the 3' end of the mRNA is polyadenylated by an enzyme such as E. coli Poly(A) polymerase after IVT.

The RNA polymerase to be added to the reaction solution for IVT may be suitably selected depending on the promoter sequence of the template DNA. For example, if the template DNA has a T7 promoter sequence, it is preferred to use a T7 RNA polymerase that recognizes that sequence. Furthermore, when the transcription template contains an SP6 or T3 promoter sequence, it is preferable to use SP6 RNA polymerase or T3 RNA polymerase, respectively.

An example of a typical IVT reaction for synthesizing mRNA of the present invention is shown below, but is not limited to the following.

1.0 µg of template DNA, 2.0 µL of 10× transfer buffer solution (400 mM Tris-HCl, pH 8.0), 190 mM MgCl₂, 50 mM DTT, 10 mM spermidine), 0.2 µL custom NTP (each 25 mM), 20U RNase inhibitor, 3000U T7 RNA polymerase, and up to 20.0 µL of dH₂O were mixed, which was then incubated at 37°C for 2 to 5 hours. The template DNA is digested using 1U RNase-free DNase on the resulting crude mixture of IVT obtained by the incubation. After incubation at 37°C for 15 minutes, mRNA is purified according to manual using Ambion's MEGAclear (registered trademark) Kit (Austin, TX), or the like. After the purification, the RNA polynucleotide is quantified using NANODROP (registered trademark) or the like, and analyzed by agarose gel electrophoresis to confirm that the RNA polynucleotide is of the proper size and no degradation of RNA has occurred.

The mRNA of the present invention may have a Cap at its 5' terminus. In IVT, a method of adding 5'-Cap to mRNA include incorporating 5'-Cap into the IVT reaction product by adding a capdinucleotide, such as N7-methyl-guanosine-5'-triphosphate-5'-guanosine, to the reaction solution of the IVT. In addition, by using capping dinucleotide analogs called Anti-Reverse Cap Analogues (ARCA) (for example, 3'-O-Mem7G(5')ppp(5')G), the Cap structure can be added to mRNA with a high probability and in the correct direction. As another embodiment, CleanCap (registered trademark) Reagent (TriLink, Inc.) can be used to add a Cap1 in which a hydroxyl group at the 2' position of the first ribose on the 5' end of the mRNA is O-methylated. As yet another method, 5'-Cap can be added to the IVT reaction product by a capping enzyme after the IVT reaction. For example, 5'-Cap can also be enzymatically added using a ScriptCap (registered trademark) m7G Capping System (Cellscript, Madison, WI, USA).

If the DNA template has no polyT, a Poly(A) tailing reaction may be performed prior to purification of the final product. If Poly(A) is already present in the transcript, the Poly(A) tailing reaction may be skipped to immediately proceed to mRNA purification.

The Poly(A) tailing reaction does not always precisely result in adenine nucleotides of a desired length, and can vary by about 10 mers from the desired length. That is, for example, when Poly(A) is described herein as 80 mers, about 60 to 100 mers, and preferably about 70 to 90 mers, 75 mers, 76 mers, 77 mers, 78 mers, 79 mers, 83 mers, 84 mers, 82 mers, 80 mers, 81 mers, or 85 mers of Poly(A) is within the scope of the mRNA of the present invention.

As a method of introducing a modified base into the mRNA produced by IVT, a modified NTP may be added in place of the NTP added to the above-mentioned IVT. For example, an mRNA containing pseudouridine (Ψ) can be produced by adding pseudouridine (Ψ)-5'-Triphosphate instead of UTP in order to replace uridine in the mRNA with pseudouridine (Ψ).

A known ligand may be attached to the polynucleotide of the present invention. Known ligands in the art can be used to enable tracking of the polynucleotide of the present invention, to improve the pharmacokinetics or pharmacodynamics of the polynucleotide of the present invention, to enhance the stability or binding affinity of the polynucleotide of the present invention, to improve intracellular behavior including cellular uptake of the polynucleotide of the present invention, or to incorporate the polynucleotide of the present invention as a component of a transport carrier composed of a single molecule or a plurality of molecules (such as liposomes, lipid nanoparticles (LNPs), polymers, micelles, or viral particles). Examples of the ligands include nucleic acids, reporter molecules, lipids (such as fatty acids, fatty chains, cholesterol, phospholipids, and the like), sugars (such as N-acetyl-galactosamine), vitamins, peptides (such as membrane-permeable peptides, cell-targeting peptides, receptor-binding peptides, endosomal escape-promoting peptides, RGD peptides, peptides having high affinity for blood components, tissue-targeting peptides, and the like), PEG (polyethylene glycol), dyes, and fluorescent molecules.

The polynucleotide of the present invention encompasses any pharmaceutically acceptable salt, ester, or salt of such an ester, or any other equivalent thereof, which, when administered to animals including humans, is capable of providing a biologically active metabolite or a residue thereof, either directly or indirectly. Accordingly, the present invention encompasses prodrugs of the polynucleotide of the present invention, pharmaceutically acceptable salts of the polynucleotide and such prodrugs, and other biologically equivalent variants thereof.

The term "prodrug" refers to an inactive or less active derivative that is converted in vivo or intracellularly into an active form (i.e., a drug) by the action of endogenous enzymes and/or other chemical substances and/or conditions. For example, the prodrug is described in T. Higuchi and V. Stella, "Pro-drugs as Novel Delivery Systems", Vol. 14 of the A.C.S. Symposium Series and Bioreversible Carriers in Drug Design, ed. Edward B. Roche, American Pharmaceutical Association and Pergamon Press, 1987.

The term "pharmaceutically acceptable salt" refers to a physiologically and pharmaceutically acceptable salt of the polynucleotide of the present invention, i.e., a salt that retains the desired biological activity of the polynucleotide and thus does not impart undesired toxicological effects.

Examples of the pharmaceutically acceptable salt include salts with alkali metals (for example, lithium, sodium, and potassium), alkaline earth metals (for example, calcium, barium, and magnesium), transition metals (for example, zinc and iron), ammonia, organic bases (for example, trimethylamine, triethylamine, dicyclohexylamine, ethanolamine, diethanolamine, triethanolamine, meglumine, diethanolamine, ethylenediamine, pyridine, picoline, and quinoline), and amino acids, or salts with inorganic acids (for example, hydrochloric acid, sulfuric acid, nitric acid, carbonic acid, hydrobromic acid, phosphoric acid, and hydroiodic acid) and organic acids (for example, formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, and ethanesulfonic acid). The pharmaceutically acceptable salt can be, particularly, a sodium salt or a potassium salt.

These salts can be formed according to methods that are conventionally carried out.

The polynucleotide of the present invention has not only HIV Tat expression activity but also usefulness as a medicine, and has any or all of the following excellent characteristics.
a) It has low inhibitory activity against CYP enzymes (for example, CYP1A2, CYP2C9, CYP2C19, CYP2D6, and CYP3A4).
b) It has high chemical stability.
c) It has no mutagenicity.
d) It has a low risk of affecting the cardiovascular system.
e) It exhibits high solubility.
f) It induces Tat antibodies with high neutralizing activity.
g) It exhibits superior antibody titers with fewer administrations than Tat protein (peptide). Specifically, two administrations of the polynucleotide of the present invention (particularly mRNA) are superior in antibody induction ability compared with three administrations of Tat protein (peptide).

The polynucleotide of the present invention may also have been modified. The suitably modified polynucleotide has any or all of the following characteristics in comparison to the unmodified polynucleotide.
a) It exhibits a sustained effect.
b) It has high resistance to nucleases.
c) It shows improved pharmacokinetics.
d) It exhibits enhanced tissue distribution.
e) It exhibits low immunogenicity.
f) It exhibits high solubility.
g) It has high chemical stability.

The polynucleotide of the present invention can be utilized in conjunction with suitable transport carriers. As a result, any or all of the following characteristics are obtained.
a) It shows highly selective migration to a target organ.
b) It efficiently enters target cells and exhibits its effect at a low dose.
c) It exhibits favorable pharmacokinetics.
d) It has higher metabolic stability.
e) It has no mutagenicity.
f) It has a low risk of affecting the cardiovascular system.
g) It exhibits high solubility.
h) It has high chemical stability.

The present invention also encompasses a pharmaceutical composition comprising the polynucleotide of the present invention. The administration method and formulation of the pharmaceutical composition of the present invention are not limited to the above-described modification method or method for adding ligands, and any administration method and formulation known in the art may be used. For example, transport carriers consisting of single or multiple multimolecules (liposomes, LNP, polymers, micelles, virus particles, and the like) can be used. In addition, polysomes, lipoplex, and the like may be used.

An example for synthesizing the pharmaceutical composition of the present invention is shown below. When a cationic lipid, a neutral lipid, a sterol, and a polyethylene glycol-modified lipid can be dissolved in a polar organic solvent, a lipid solution can be obtained. When an mRNA solution of the present invention prepared using a buffer solution and the lipid solution are mixed, a nucleic acid-lipid mixture solution can be obtained. By replacing the polar organic solvent in the polar organic solvent with an aqueous solution, such as a buffer solution, the pharmaceutical composition of the present invention can be obtained.

For a general description of the method for manufacturing the pharmaceutical composition of the present invention, see Remington: The Science and Practice of Pharmacy, Twenty third Edition, 2020, and the like.

The pharmaceutical composition of the present invention can be administered by various methods, depending on whether local or systemic treatment is desired, or on the area to be treated. Examples of the administration method includes local administrations (including ocular, vaginal, rectal, intranasal, and transdermal administrations), oral administration, and parenteral administration. The parenteral administration can be intramuscular, intraperitoneal, or subcutaneous injection; intravenous injection or infusion; pulmonary administration by aspiration or inhalation; intrathecal administration; or intraventricular administration. The parenteral administration is preferably intramuscular injection.

When the pharmaceutical composition of the present invention is administered locally, a formulation such as a transdermal patch, ointment, lotion, cream, gel, a dripping agent, a suppository, a propellant, a solution, and a powder can be used.

The composition for oral administration can be a powder, a granule, a suspension or solution in water or non-aqueous media, a capsule, a powder, a tablet, or the like.

The composition for parenteral, subdural, or intraventricular administration can be a sterile aqueous solution or the like that contains a buffer, a diluent, and other suitable additives.

The pharmaceutical composition of the present invention can be obtained by mixing, as necessary, an effective amount of the polynucleotide of the present invention with various pharmaceutical additives appropriate for the dosage form, such as a stabilizer, a solubilizer, an excipient, a tonicity agent, or a pH adjuster. In the case of injection, it may be formulated by sterilization together with an appropriate carrier.

The stabilizer can be purified white sugar, trehalose, maltose, glucose, sodium edetate, sodium chloride, amino acid, or the like. The solubilizer can be polysorbate 20, polysorbate 80, polyoxyethylene-cured castor oil, poloxamer, or the like. The excipient can be maltose, xylitol, sodium chloride, amino acid, purified white sugar, trehalose, maltose, or the like. The tonicity agent can be sodium chloride, glycerin, glucose, xylitol, or the like. As the pH adjuster, hydrochloric acid, sodium hydroxide, sodium hydrogenphosphate, sodium dihydrogenphosphate, citric acid, sodium citrate, acetic acid, sodium acetate, trometamol, trometamol hydrochloride, or the like can be used. When the pharmaceutical composition is formulated as a liquid or as an emulsion or suspension for injection, commonly used solubilizing agents, suspending agents, emulsifiers, stabilizers, preservatives, tonicity agents, and the like may be added as appropriate. In the case of oral administration, a charming agent, a fragrance, or the like may be added.

The administration depends on the severity and responsiveness of the condition being treated, and the course of treatment may continue for several days to several months, or until cure is achieved or amelioration of the condition is attained. The optimal administration schedule can be calculated based on measurements of drug accumulation in a biological body. A person skilled in the art can determine the optimal dose, route of administration, and frequency of administration. The optimal dosage varies according to the relative potency of each nucleic acid, and can generally be calculated based on antibody titers, IC50, or EC50 in in vitro and in vivo animal experiments. For example, given the molecular weight of a polynucleotide (derived from its nucleic acid sequence and chemical structure) and an effective dose, such as an antibody titer (determined experimentally), a dosage expressed in mg/human can be calculated in accordance with conventional practice. When the pharmaceutical composition of the present invention is administered to humans, it may be administered at a dose of about 0.001 to 1 mg, preferably 0.01 to 0.2 mg, of mRNA per adult per administration, once or multiple times, by intramuscular injection, subcutaneous injection, intradermal injection, intravenous infusion, or intravenous injection; however, the dose and number of administrations may be appropriately varied depending on the type of disease, symptoms, age, route of administration, and the like.

The pharmaceutical composition of the present invention can be used as a therapeutic and/or prophylactic agent for a HIV-associated disease. The HIV-associated disease means HIV infection, opportunistic infection selected from candidiasis, coccidioides, cryptococcus disease, cryptosporidiosis, cytomegalovirus, herpes simplex virus, herpes zoster, histoplasmosis, isosporosis, mycobacterium avium complex, Pneumocystis pneumonia, bacterial pneumonia, progressive multifocal leukoencephalopathy Salmonella, Toxoplasmosis, and tuberculosis, AIDS-related cancers selected from cervical cancer, Kaposi's sarcoma, and lymphoma, candidiasis of the esophagus, bronchi, trachea, or lungs, invasive cervical cancer, disseminated or extrapulmonary coccidioidomycosis, extrapulmonary cryptococcosis, chronic intestinal cryptosporidiosis, cytomegalovirus disease (excluding liver, spleen, or lymph nodes), cytomegalovirus retinitis with vision loss, HIV-associated encephalopathy, and herpes simplex virus infection (with chronic ulcers, bronchitis, interstitial pneumonia, or esophagitis), disseminated or extrapulmonary histoplasmosis, chronic intestinal isosporiasis, Kaposi's sarcoma, Burkitt's lymphoma, immunoblastic lymphoma, primary cerebral lymphoma, disseminated or extrapulmonary Mycobacterium avium complex or Candida infections, pulmonary or extrapulmonary tuberculosis, disseminated or extrapulmonary mycobacterial infections, pneumocystis jirovecii pneumonia, recurrent pneumonia, progressive multifocal leukoencephalopathy, recurrent Salmonella septicemia, cerebral toxoplasmosis, and HIV-associated wasting syndrome, AIDS dementia complex, AIDS-induced encephalopathy, HIV encephalopathy, HIV-associated progressive encephalopathy, HIV-associated neurocognitive disorder (HAND), asymptomatic neurocognitive impairment (ANI), mild neurocognitive disorder (MND), HIV-associated dementia (HAD), mild cognitive-motor disorder (MCMD), vacuolar myelopathy, peripheral neuropathy, and polymyositis, or the like. The HIV-associated disease is particularly preferably HIV infection.

### Examples

Hereinafter, the present invention will be described in more detail by way of examples and reference examples; however, the present invention is not intended to be limited by these.

### Example 1 Preparation of template DNA for IVT of Tat mRNA

The plasmid DNA was amplified and purified by PCR to prepare the template DNA used for IVT. The DNA fragment comprising a sequence sequentially linked by a T7 promoter sequence, 5'-UTR sequence, ORF, and 3'-UTR sequence was introduced into plasmid. 2×Prime STAR (registered trademark) MAX DNA polymerase Premix (Takara catalog #R045), 10 µM sense primer (SEQ ID NO: 180), and 10 µM antisense primer (SEQ ID NO: 181) were added to the Nuclease-free water in which the plasmid was lysed, which was then incubated at 98°C for 2 minutes, and then subjected to 35 cycles of incubating at 98°C for 10 seconds, at 55°C for 5 seconds, and at 72°C for 10 seconds to amplify DNA. After the reaction, the template DNA was purified by NucleoSpin (registered trademark) Gel and PCR Clean-up (Takara catalog #740609.250).

### Example 2 Preparation of Tat mRNA by IVT

The template DNA obtained in Example 1, 75 mM ATP, 75 mM GTP, 75 mM CTP or 100 mM 5-Methyl-CTP, 100 mM N1-Methylpseudo-UTP or 100 mM 5-Methyl-UTP, Nuclease-free water, 10X Reaction Buffer (Thermo Fisher catalog #AMB13345), and Enzyme mix T7 RNA Polymerase (Thermo Fisher catalog #AMB13345) were mixed and incubated at 37°C for 2 hours. TURBO DNase (Thermo Fisher catalog #AM2239) was mixed and incubated at 37°C for 15 min.

A 7.5 M LiCl solution was mixed and allowed to stand at -20°C overnight. After centrifugation, the supernatant was discarded. 70% ethanol was added thereto, and after centrifugation, the supernatant was discarded and air-dried. The resulting residue was dissolved in Nuclease-free water and then a portion of the resulting solution was mixed with Nuclease-free water, 10 mM GTP, 20 mM SAM, 10X Capping Buffer (New England Biolab catalog #M2080), Vaccinia Capping Enzyme (10U/µL, New England Biolab catalog #M2080), mRNA Cap2'-O-Methyltransferase (50U/µL, New England Biolab catalog #M0366), which was then incubated at 37°C for 2 hours. A 7.5M LiCl solution was mixed and allowed to stand at -20°C overnight. After centrifugation, the supernatant was discarded, 70% ethanol was added, and after centrifugation, the supernatant was discarded and air-dried. The resulting residue was dissolved in Nuclease-free water, and the resulting solution was mixed with 10X Antarctic Phosphatase Reaction Buffer (New England Biolab catalog #B0289), Antarctic Phosphatase (10 µL, New England Biolab catalog #M0289), which was then incubated at 37°C for 30 minutes. The intended mRNA was obtained by purification according to the accompanying manual using a Monarch RNA Cleanup Kit (New England Biolab catalog #T2050). The obtained mRNA was analyzed by TapeStation (Agilent) to confirm the desired length.

### Example 3 Tat mRNA Synthesis

The methods of Examples 1 and 2 were used to synthesize HIV Tat mRNA with 5' UTR, ORF, 3' UTR, Poly(A), and modified bases as described in Tables 1, 2-1 and 2-2. The 5' Cap structure of each Tat mRNA is Cap1, represented as 5'-[m7G]-ppp-GmGG. Gm indicates guanosine in which the 2' position is O-methylated. Specifically, this is represented by the following structure. By using CleanCap (registered trademark) Reagent AG and CleanCap (registered trademark) Reagent AU (both TriLink) and the like, HIV Tat mRNA having Capl represented as 5'-[m7G]-ppp-AmGG or 5'-[m7G]-ppp-AmUA was synthesized.

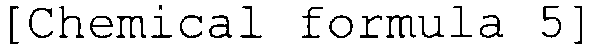

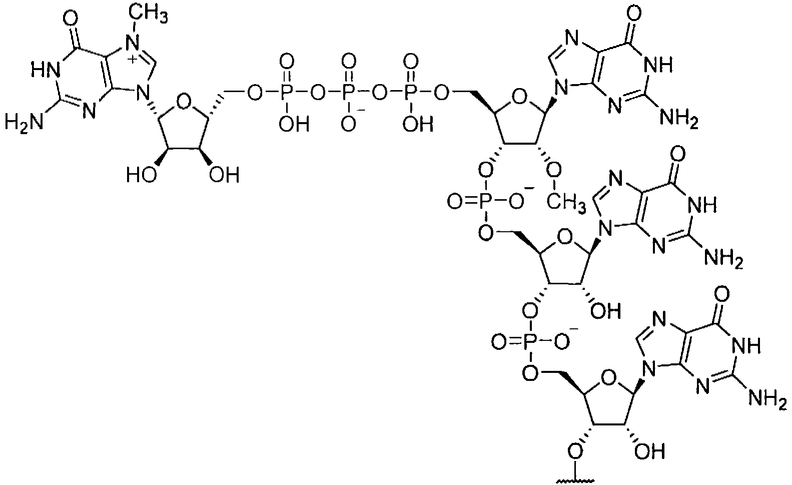

Any Tat mRNA has replaced all uridine bases in the sequence by an N1-methylpseudouridine (m1Ψ) base or a 5-methyluridine (5MeU) base, and in some sequences all cytidine bases in the sequence by a 5-methylcytidine (5MeC) base. Substituted modified bases are described in the Modified bases in the tables. Except SNG-330 and SNG-337, they encode a secretory signal peptide and the Tat protein and the peptide name and the amino acid sequence numbers are listed in the peptide column within the tables. It should be noted that the nucleic acid sequences of mRNA other than SNG-299 have been optimized for the codon of the secretory signal peptide and the codon of Tat, respectively. For example, SNG-331 represents mRNA encoding the Igk HIV-Tat BH10 protein (SEQ ID NO: 168), with the secretory signal peptide Igk (SEQ ID NO: 162) linked to the N-terminus of the HIV-Tat BH10 protein (SEQ ID NO: 134). SNG-331 is an mRNA consisting of, in the 5' to 3' direction, a 5' Cap structure consisting of 5'-[m7G]-ppp-GmGG, a 5' UTR represented by SEQ ID NO: 1, an ORF represented by SEQ ID NO: 114, a 3' UTR represented by SEQ ID NO: 64, and a Poly(A) of about 80 mers, all uridine bases are m1Ψ. SNG-346 and SNG-347 represent mRNAs encoding an IgE HIV-Tat Oyi MITD protein (SEQ ID NO: 191) or an IgE HIV-Tat Oyi Foldon protein (SEQ ID NO: 192), in which a secretory signal peptide IgE (SEQ ID NO: 161) is linked to the N-terminus of the HIV-Tat Oyi protein (SEQ ID NO: 133), and a secretory signal peptide MITD (SEQ ID NO: 188) or Foldon (SEQ ID NO: 189) is further linked to the C-terminus thereof. Other tables are listed similarly.

### [Table 1]

**Table 1**

| SNG No. | Amino acid sequence | | | | | Nucleotide sequence | | | | | |
|---|---|---|---|---|---|---|---|---|---|---|---|
| | peptide | | Tat | | ORF SEQ ID | Cap | 5' UTR SEQ ID | ORF SEQ ID | 3' UTR SEQ ID | Poly (A) (mer) | Modified base |
| | name | SEQ ID | name | SEQ ID | | | | | | | |
| 330 | - | - | BH10 | 134 | 134 | Capl | 1 | 113 | 64 | 80 | m1Ψ |
| 331 | Igk | 162 | BH10 | 134 | 168 | Cap1 | 1 | 114 | 64 | 80 | m1Ψ |
| 332 | JEV | 163 | BH10 | 134 | 169 | Cap1 | 1 | 115 | 64 | 80 | m1Ψ |
| 333 | SARS | 164 | BH10 | 134 | 170 | Cap1 | 1 | 116 | 64 | 80 | m1Ψ |
| 334 | IgG | 167 | BH10 | 134 | 171 | Cap1 | 1 | 117 | 64 | 80 | m1Ψ |
| 335 | tPA | 165 | BH10 | 134 | 172 | Cap1 | 1 | 118 | 64 | 80 | m1Ψ |
| 336 | albumin | 166 | BH10 | 134 | 173 | Cap1 | 1 | 119 | 64 | 80 | m1Ψ |

### [Table 2-1]

**Table 2-1**

| SNG No. | Amino acid sequence | | | | | Nucleotide sequence | | | | | |
|---|---|---|---|---|---|---|---|---|---|---|---|
| | peptide | | Tat | | ORF SEQ ID | Cap | 5' UTR SEQ ID | ORF SEQ ID | 3' UTR SEQ ID | Poly (A) (mer) | Modified base |
| | name | SEQ ID | name | SEQ ID | | | | | | | |
| 337 | - | - | Oyi | 133 | 133 | Cap1 | 1 | 120 | 64 | 80 | m1Ψ |
| 327 | IgE | 161 | Oyi | 133 | 174 | Capl | 1 | 121 | 64 | 80 | m1Ψ |
| 338 | Igk | 162 | Oyi | 133 | 175 | Capl | 1 | 122 | 64 | 80 | m1Ψ |
| 339 | JEV | 163 | Oyi | 133 | 176 | Capl | 1 | 123 | 64 | 80 | m1Ψ |
| 340 | SARS | 164 | Oyi | 133 | 177 | Cap1 | 1 | 124 | 64 | 80 | m1Ψ |
| 341 | IgG | 167 | Oyi | 133 | 178 | Cap1 | 1 | 125 | 64 | 80 | m1Ψ |
| 345 | IgE | 161 | Oyi | 133 | 174 | Cap1 | 3 | 121 | 66 | 80 | m1Ψ |
| 329 | IgE | 161 | Oyi | 133 | 174 | Capl | 2 | 126 | 65 | 80 | m1Ψ |
| 299 | IgE | 161 | Mod-Oyi | 182 | 190 | Capl | 1 | 198 | 64 | 80 | m1Ψ |
| 322 | IgE | 161 | Mod-Oyi | 182 | 190 | Capl | 1 | 199 | 64 | 80 | m1Ψ |
| 323 | IgE | 161 | Mod-Oyi | 182 | 190 | Capl | 1 | 200 | 64 | 80 | m1Ψ |
| 324 | IgE | 161 | Mod-Oyi | 182 | 190 | Capl | 1 | 201 | 64 | 80 | m1Ψ |
| 325 | IgE | 161 | Oyi | 133 | 174 | Capl | 1 | 202 | 64 | 80 | m1Ψ |
| 328 | IgE | 161 | Oyi | 133 | 174 | Capl | 1 | 203 | 64 | 80 | m1Ψ |
| 346 | IgE MITD | 161 188 | Oyi | 133 | 191 | Cap1 | 1 | 204 | 64 | 80 | m1Ψ |
| 347 | IgE Foldon | 161 189 | Oyi | 133 | 192 | Capl | 1 | 205 | 64 | 80 | m1Ψ |
| 348 | Cystatin S | 183 | Oyi | 133 | 193 | Cap1 | 1 | 206 | 64 | 80 | m1Ψ |
| 349 | Mod-tPA | 184 | Oyi | 133 | 194 | Cap1 | 1 | 207 | 64 | 80 | m1Ψ |
| 350 | IL-6 | 185 | Oyi | 133 | 195 | Cap1 | 1 | 208 | 64 | 80 | m1Ψ |
| 351 | CTLA4 | 186 | Oyi | 133 | 196 | Cap1 | 1 | 209 | 64 | 80 | m1Ψ |
| 352 | HSV/gDsec | 187 | Oyi | 133 | 197 | Capl | 1 | 210 | 64 | 80 | m1Ψ |
| 359 | - | - | Oyi | 133 | 133 | Cap1 | 1 | 120 | 64 | 80 | 5MeU |
| 360 | IgE | 161 | Oyi | 133 | 174 | Cap1 | 1 | 121 | 64 | 80 | 5MeU |
| 361 | Igk | 162 | Oyi | 133 | 175 | Cap1 | 1 | 122 | 64 | 80 | 5MeU |
| 362 | JEV | 163 | Oyi | 133 | 176 | Capl | 1 | 123 | 64 | 80 | 5MeU |
| 363 | SARS | 164 | Oyi | 133 | 177 | Cap1 | 1 | 124 | 64 | 80 | 5MeU |

### [Table 2-2]

**Table 2-2**

| SNG No. | Amino acid sequence | | | | | Nucleotide sequence | | | | | |
|---|---|---|---|---|---|---|---|---|---|---|---|
| | peptide | | Tat | | ORF SEQ ID | Cap | 5' UTR SEQ ID | ORF SEQ ID | 3' UTR SEQ ID | Poly (A) (mer) | Modified base |
| | name | SEQ ID | name | SEQ ID | | | | | | | |
| 364 | IgG | 167 | Oyi | 133 | 178 | Capl | 1 | 125 | 64 | 80 | 5MeU |
| 365 | IgE | 161 | Mod-Oyi | 182 | 190 | Capl | 1 | 198 | 64 | 80 | 5MeU |
| 366 | - | - | Oyi | 133 | 133 | Cap1 | 1 | 120 | 64 | 80 | 5MeU, 5MeC |
| 367 | IgE | 161 | Oyi | 133 | 174 | Capl | 1 | 121 | 64 | 80 | 5MeU, 5MeC |
| 368 | Igk | 162 | Oyi | 133 | 175 | Capl | 1 | 122 | 64 | 80 | 5MeU, 5MeC |
| 369 | JEV | 163 | Oyi | 133 | 176 | Capl | 1 | 123 | 64 | 80 | 5MeU, 5MeC |
| 370 | SARS | 164 | Oyi | 133 | 177 | Capl | 1 | 124 | 64 | 80 | 5MeU, 5MeC |
| 371 | IgG | 167 | Oyi | 133 | 178 | Cap1 | 1 | 125 | 64 | 80 | 5MeU, 5MeC |
| 372 | IgE | 161 | Mod-0yi | 182 | 190 | Capl | 1 | 198 | 64 | 80 | 5MeU, 5MeC |

In addition, SIV Tat mRNA with the structure shown in Table 3 and in which all uridine bases in the sequence were replaced by N1-methylpseudouridine (m1Ψ) base was purchased from TriLink.

### [Table 3]

**Table 3**

| SNG No. | Amino acid sequence | | | | Nucleotide sequence | | | |
|---|---|---|---|---|---|---|---|---|
| | peptide | | Tat SEQ ID | ORF SEQ ID | Cap | ORF SEQ ID | Poly (A) (mer) | Modified base |
| | name | SEQ ID | | | | | | |
| 0 | - | - | 160 | 160 | Capl | 127 | 120 | m1Ψ |
| 3 | IgE | 161 | 160 | 179 | Capl | 138 | 120 | m1Ψ |

### Example 4 Construction of ELISA System for Measurement of Tat Protein of HIV-1

Anti HIV-1 Tat monoclonal antibodies (Abcam) were immobilized on 384-well flat bottom plate for ELISA to prepare anti-Tat antibody solid phase plate. The ELISA system for measurement of Tat protein was constructed using the combination of biotin-labeled anti-HIV-1 Tat polyclonal antibody (Abcam), horseradish peroxidase-labeled streptavidin (Thermo Fisher Scientific), and SuperSignal ELISA Pico Chemiluminescent Substrate (Thermo Fisher Scientific).

### Example 5 Evaluation of Tat Expression in Cultured Cell Lines with Tat mRNA

Tat protein expression ability in cultured cell lines was evaluated using the mRNA synthesized in Example 3. A human cervical carcinoma-derived cell line HeLa was suspended in DMEM medium containing 10% fetal bovine serum (FBS), 100 units/ml penicillin, and 0.10 mg/ml streptomycin at 10,000 cells/90 µL. The cells were seeded at 90 µL per well in a 96-well flat-bottom plate and cultured for one day at 37°C under 5%CO₂ and 95 to 98% humidity. On the following day, 100 ng of each mRNA listed in Table 3 was diluted in 5 µL of OPTI-MEM (Thermo Fisher Scientific). Lipofectamine MessengerMAX Transfection Reagent (Thermo Fisher Scientific) was used as a transfection reagent and was diluted in 5 µL of OPTI-MEM. The amount of transfection reagent added was prepared such that 0.2 µL per 100 ng of mRNA. After incubation at room temperature for 10 minutes, the two solutions were mixed and further incubated for 5 minutes. The mixed solution was diluted with OPTI-MEM to give an mRNA concentration of 25 ng/10 µL, and 10 µL of the diluted solution was added to the cell culture plate. Thereafter, the cells were continuously cultured at 37°C. The test was performed under each condition N=2 or 3.

After washing with PBS, HeLa cells were lysed with 100 µL of RIPA Lysis and Extraction Buffer (Thermo Fisher Scientific) supplemented with a protease inhibitor cocktail (Merck). The concentration (ng/ml) of Tat protein in the collected cell lysates was quantified using the ELISA system described in Example 4 (Tables 4-1 to 4-4). The leftmost column of Tables 4-1 to 4-4 shows the numbers of the control and mRNA used. Each of the mRNAs were confirmed to express the Tat protein.

### [Table 4-1]

**Table 4-1**

| SNG No | mRNA | Tat (ng/ml) |
|---|---|---|
| 330 | Tat BH10 | 328.57 |
| 331 | Igk Tat BH10 | 449.82 |
| 332 | JEV Tat BH10 | 228.68 |
| 333 | SARS Tat BH10 | 566.43 |
| 334 | IgG Tat BH10 | 122.7 |
| 335 | tPA Tat BH10 | 341.76 |
| 336 | albumin Tat BH10 | 835.71 |

### [Table 4-2]

**Table 4-2**

| SNG No | mRNA | Tat (ng/ml) |
|---|---|---|
| 337 | Tat Oyi | 176.92 |
| 327 | IgE Tat Oyi | 48.79 |
| 338 | Igk Tat Oyi | 56.68 |
| 339 | JEV Tat Oyi | 40.1 |
| 340 | SARS Tat Oyi | 34.05 |
| 341 | IgG Tat Oyi | 47.61 |
| 345 | IgE Tat Oyi | 44.2 |

### [Table 4-3]

**Table 4-3**

| SNG No | mRNA | Tat (ng/ml) |
|---|---|---|
| 327 | IgE Tat Oyi | 197.4 |
| 359 | Tat Oyi | 179.6 |
| 360 | IgE Tat Oyi | 139.3 |
| 361 | Igk Tat Oyi | 160.8 |
| 362 | JEV Tat Oyi | 160.5 |
| 363 | SARS Tat Oyi | 155.4 |
| 364 | IgG Tat Oyi | 163.5 |
| 365 | IgE Mod-Tat Oyi | 203.5 |
| 366 | Tat Oyi | 146.5 |
| 367 | IgE Tat Oyi | 129.7 |
| 368 | Igk Tat Oyi | 145.9 |
| 369 | JEV Tat Oyi | 162.8 |
| 370 | SARS Tat Oyi | 156.0 |
| 371 | IgG Tat Oyi | 149.9 |
| 372 | IgE Mod-Tat Oyi | 138.2 |

### [Table 4-4]

**Table 4-4**

| SNG No | mRNA | Tat (ng/ml) |
|---|---|---|
| 346 | IgE Tat Oyi MITD | 199.7 |
| 347 | IgE Tat Oyi Foldon | 111.9 |
| 348 | Cystatin S Tat Oyi | 102.3 |
| 349 | Mod-tPA Tat Oyi | 99.4 |
| 350 | IL-6 Tat Oyi | 133.6 |
| 351 | CTLA4 Tat Oyi | 118.7 |
| 352 | HSV/gDsec Tat Oyi | 126.7 |

### Example 6 Preparation of mRNA-Encapsulated Nucleic Acid Lipid Particles with Tat mRNA

The cationic lipid described in WO 2022/168884 (hereinafter referred to as "Lipid"), distearoylphosphatidylcholine (1,2-Distearoyl-sn-glycero-3-phosphocholine; hereinafter referred to as "DSPC", NOF CORPORATION), Cholesterol (hereinafter referred to as "Chol", NIPPON FINE CHEMICAL CO., LTD.), and 1,2-Dimyristoyl-sn-Glycero-3-Methoxypolyethylene Glycol having a polyethylene glycol molecular weight of about 2000 (hereinafter referred to as "PEG-DMG", NOF CORPORATION) were dissolved in ethanol at a molar ratio of DSPC:Chol:Lipid:PEG-DMG=8.4:36:54:1.6 to give a total lipid concentration of 33 mM, thereby obtaining a lipid solution.

On the other hand, the Tat mRNA synthesized in Example 3 was prepared at a concentration of 292 µg/mL in a citrate buffer solution (pH 4.0) to obtain an mRNA solution.

The lipid solution and the mRNA solution described above were mixed in a microfluidic channel using a NanoAssemblr IGNITE (Precision Nanosystems Inc.) at a volume ratio of 1:3 to obtain a crude dispersion of lipid-nucleic acid particles.

The crude dispersion of lipid-nucleic acid particles described above and a phosphate buffer solution (pH 7.5) were subjected to in-line dilution at a volume ratio of 2:1 (the microchannel was an NxGen Mixer Dilution microfluidic cartridge for IGNITE), to obtain a dispersion of lipid-nucleic acid particles. The dispersion of the nucleic acid-lipid particles was dialyzed against about 100 volumes of 20 mM Tris-HCl buffer solution for 3 hours, and then further dialyzed against about 100 volumes of 20 mM Tris-HCl buffer solution for 12 to 18 hours (using Slide-A-Lyzer Dialysis Cassette G2, MWCO: 10 kD, Spectra/Por) to remove ethanol. The dialyzed dispersion was concentrated using amicon (Amicon Ultra-15 PLHK Ultracel-PL membrane, MWCO 100 kDa, Merck) and adjusted to the desired concentration with 20 mM Tris-HCl buffer solution. The concentrated dispersion was then mixed with an additive stock solution (prepared by mixing the dispersion with 20 mM Tris-HCl buffer solution and 1.05M sucrose at a volume ratio of 1:2), yielding a dispersion of purified mRNA-encapsulated nucleic acid-lipid particles in 20 mM Tris-HCl buffer solution containing 350 mM sucrose.

### Example 7 Evaluation of Antibody Inducing Ability of Tat mRNA using Mice

The RNA vaccine containing 25 µg of mRNA per mouse was injected into the thigh muscle of the hind limb twice at three-week intervals. Two weeks after the second immunization, whole blood was collected from the abdominal vena cava using a syringe with a needle containing 10 µL of heparin, and then centrifuged in a refrigerated centrifuge (20,400 g, 5 min, 4°C) to obtain plasma. The anti-Tat antibody titer in the plasma was measured by ELISA. Tat Oyi Protein 101aa (Peptide Institute, Inc.) was prepared in PBS at pH 7.4 at a concentration of 1 µg/mL. The prepared solution was dispensed at 25 µL per well into the plate, sealed, and left to stand overnight at 4°C. The next day, the antigen solution was removed from the plate, and 90 µL of wash buffer (PBS-T) was added per well and washed twice. To the washed plate, 50 µL per well of blocking buffer (1% BSA/PBS) was added and left to stand at room temperature. After 1 hour, the blocking buffer was removed from the plate, 90 µL of wash buffer was added per well, and the plate was washed three times. The washed plate was treated by adding 25 µL per well of plasma diluted with dilution buffer (1% BSA/PBS) in duplicate (N=2) and left to stand at room temperature. After 2 hours, the plasma samples were removed from the plate, and the wells were washed three times with 90 µL of wash buffer per well. The washed plate was then left to stand at room temperature with 25 µL per well of HRP-labeled goat anti-mouse IgG Fc secondary antibody (Jackson Immuno Research) diluted appropriately. After 1 hour, the secondary antibody solution was removed from the plate, and the wells were washed three times with 90 µL of wash buffer per well. Then, 25 µL of TMB solution was added to each well of the washed plate, which was left to stand at room temperature. The reaction was stopped by adding 25 µL of 2N sulfuric acid per well after 10 minutes. The absorbance at 450 nm was measured using a spectrophotometer. Anti-Tat antibody titers were calculated using XLfit (registered trademark) or a program with equivalent computational capability, according to the following method. The absorbance of wells containing only the dilution buffer was used as the blank, and the cut-off value was set to be the average absorbance of the blank plus 0.5. In the dilution series of plasma samples, the dilution factor at which the mean absorbance (N=2) corresponded to the cut-off value was calculated as the anti-Tat antibody titer, and the geometric mean titer (GMT) for each group was determined. Results are shown in Tables 5, 6-1 and 6-2. If the absorbance was below the cut-off value at all dilution factors, the antibody titer was assigned as the lowest dilution factor.

Compared to mRNA encoding only the HIV Tat protein, the mRNA of the present invention exhibited higher anti-Tat antibody titers (Tables 5 and 6-1). Furthermore, all mRNAs of the present invention showed excellent anti-Tat antibody titers (Table 6-2).

Using the same method, 30 µg per mouse of Tat BH10 protein (SEQ ID NO: 134) or Tat Oyi protein (SEQ ID NO: 133) was administered into the hind thigh muscles of mice three times at 4-week intervals, and the anti-Tat antibody titers in plasma were measured two weeks after the second and third immunizations (Table 6-3). It was suggested that two administrations of the mRNA of the present invention induced higher anti-Tat antibody titers than three administrations of the Tat protein (Tables 5, 6-1, 6-2, and 6-3).

### [Table 5]

**Table 5**

| SNG No. | mRNA | Anti-Tat IgG Titer (GMT) (×10⁵) after TWO immunizations |
|---|---|---|
| 330 | Tat BH10 | 1.5 |
| 331 | Igk Tat BH10 | 5.1 |
| 332 | JEV Tat BH10 | 5.1 |
| 333 | SARS Tat BH10 | 3.5 |
| 334 | IgG Tat BH10 | 13.4 |
| 335 | tPA Tat BH10 | 4.3 |
| 336 | albumin Tat BH10 | 4.3 |

### [Table 6-1]

**Table 6-1**

| SNG No. | mRNA | Anti-Tat IgG Titer (GMT) (×10⁵) after TWO immunizations |
|---|---|---|
| 337 | Tat Oyi | 0.06 |
| 327 | IgE Tat Oyi | 1.7 |
| 338 | Igk Tat Oyi | 3.9 |
| 339 | JEV Tat Oyi | 31.0 |
| 340 | SARS Tat Oyi | 10.5 |
| 341 | IgG Tat Oyi | 11.7 |
| 345 | IgE Tat Oyi | 8.4 |

### [Table 6-2]

**Table 6-2**

| SNG No. | mRNA | Anti-Tat IgG Titer (GMT) (×10⁵) after TWO immunizations |
|---|---|---|
| 325 | IgE Tat Oyi | 6.3 |
| 327 | IgE Tat Oyi | 12.9 |
| 328 | IgE Tat Oyi | 26.2 |
| 299 | IgE Mod-Tat Oyi | 10.6 |
| 322 | IgE Mod-Tat Oyi | 4.2 |
| 323 | IgE Mod-Tat Oyi | 8.3 |
| 324 | IgE Mod-Tat Oyi | 9.0 |

### [Table 6-3]

**Table 6-3**

| | Anti-Tat IgG Titer (GMT) (×10⁵) after TWO immunizations | Anti-Tat IgG Titer (GMT) (×10⁵) after THREE immunizations |
|---|---|---|
| Tat BH10 protein | 1.2 | 2.2 |
| Tat Oyi protein | 0.5 | 1.0 |

### Reference Example 1 Evaluation of Antibody Inducing Ability of SIV-Tat mRNA Using Mice

The RNA vaccine containing 25 µg of mRNA per mouse was injected into the thigh muscle of the hind limb twice at three-week intervals. Two weeks after the second immunization, blood was collected from the tail vein using a heparin-treated capillary, and the samples were centrifuged in a refrigerated centrifuge (20,400 g, 5 min, 4°C) to obtain plasma. Alternatively, mice were anesthetized with isoflurane and subjected to laparotomy, and blood was collected from the abdominal vena cava using a syringe with a needle. The collected blood was left to stand at room temperature for 4 to 5 hours and then centrifuged using a refrigerated centrifuge (200 g, 15 min, 0°C) to obtain the serum. The anti-SIV-Tat antibody titers in plasma or serum were measured by ELISA. SIV-Tat Protein (Creative Diagnostics) was prepared in PBS (pH 7.4) at a concentration of 1 µg/mL. The prepared solution was dispensed at 25 µL per well into the plate, the plate was sealed, which was then left to stand overnight at 4°C. The next day, the antigen solution was removed from the plate, and 80 µL of washing buffer (0.05% Tween-20/1% BSA/PBS) was added per well, followed by washing three times. Then, 75 µL of blocking buffer (1% BSA/PBS) was added to each well of the washed plate, which was then left to stand at room temperature. After 1 hour, the blocking buffer was removed from the plate, and 80 µL of washing buffer was added per well, followed by washing three times. The washed plate was then left to stand at room temperature with 25 µL per well of blood samples diluted in dilution buffer (1% BSA/PBS), in duplicate (N=2). After 2 hours, the blood samples were removed from the plate, and 80 µL of washing buffer was added per well, followed by three washes. The washed plate was then incubated at room temperature with 25 µL per well of diluted HRP-conjugated goat anti-mouse IgG Fc antibody (Jackson Immuno Research) as the secondary antibody. After 1 hour, the secondary antibody solution was removed from the plate, and 80 µL of washing buffer was added per well, followed by washing three times. The washed plate was then left to stand at room temperature with 25 µL per well of ELISA Pico Chemiluminescent Substrate solution (Thermo Scientific). After 5 minutes, the luminescent signal was measured with a luminescence meter.

The anti-Tat antibody titers were calculated in the following manner using a program with comparable computing capability such as XLfit. The luminescent signal of a well to which only the dilution buffer was added was determined as a blank, and a value obtained by adding 100000 to the mean value of the blank was set as the cut-off value. In dilution series of blood samples, the dilution factor was calculated as the anti-SIV-Tat antibody titer where the average of the luminescent signal of N=2 represents a cut-off value, and the GMT for each group was calculated. Table 7 shows the results. The minimum dilution factor was antibody titer when the luminescent signal fell below the cut-off value in all dilution magnifications. The mRNA encoding SIV Tat protein and secretory signal peptide showed higher anti-Tat antibody titers compared to mRNA encoding only SIV Tat protein (Table 7). Thus, it is suggested that mRNA encoding a secretory signal peptide and a Tat protein such as HIV-2 Tat that is similar in amino acid sequence to SIV Tat has similar effects.

### [Table 7]

**Table 7**

| SNG No. | mRNA | Anti-SIV-Tat IgG Titer (GMT) (×10⁵) |
|---|---|---|
| 0 | SIV-Tat | 0.001 |
| 3 | IgE SIV-Tat | 1.3 |

### Industrial Applicability

As is apparent from the above, the polynucleotide of the present invention, which encodes (a) the HIV Tat protein or its antigenic fragment and (b) a polypeptide comprising a signal peptide, can efficiently express the HIV Tat protein or its antigenic fragment and exhibits excellent ability to induce anti-Tat antibodies. Therefore, the pharmaceutical composition of the present invention is highly useful as a pharmaceutical for the treatment and/or prevention of HIV-related diseases (for example, HIV infection, HAND, and the like).

## Claims

1. A polynucleotide encoding a polypeptide comprising the following (a) and (b):
(a) an HIV Tat protein or antigenic fragment thereof; and
(b) a secretory signal peptide.

2. The polynucleotide according to claim 1, wherein (b) is (i) or (ii) :
(i) a polypeptide consisting of an amino acid sequence set forth in any one of SEQ ID NOs: 161 to 167 and 183 to 187; or
(ii) a polypeptide consisting of an amino acid sequence in which one or more amino acids are deleted, substituted, inserted, or added in an amino acid sequence set forth in any one of SEQ ID NOs: 161 to 167 and 183 to 187 and having secretory signal activity.

3. The polynucleotide according to claim 1 or 2, wherein (a) is an HIV Tat protein.

4. The polynucleotide according to any one of claims 1 to 3, wherein the HIV Tat protein is a Tat protein of HIV-1.

5. The polynucleotide according to claim 4, wherein the HIV-1 Tat protein is a polypeptide consisting of an amino acid sequence that is identical to, or having 90% or greater identity with, the amino acid sequence set forth in any one of SEQ ID NOs: 131 to 148 and 182.

6. The polynucleotide according to any one of claims 1 to 5, wherein the polypeptide comprising (a) and (b) is a polypeptide consisting of an amino acid sequence set forth in any one of SEQ ID NOs: 168 to 178 and 190 to 197.

7. The polynucleotide according to any one of claims 1 to 6, wherein the polynucleotide is an mRNA comprising an open reading frame encoding a polypeptide comprising the following (a) and (b):
(a) an HIV Tat protein or antigenic fragment thereof; and
(b) a secretory signal peptide.

8. The polynucleotide according to claim 7, further comprising: a modified base selected from 5-methyluridine, pseudouridine, N1-methylpseudouridine, 5-methoxyuridine, 2-thiouridine, 6-methyladenosine, 2-aminoadenosine, inosine, 5-methylcytidine, N1-ethylpseudouridine, 4-thiouridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxypseudouridine, 4-thio-1-methyl-pseudouridine, 4-thio-pseudouridine, 5-aza-uridine, dihydropseudouridine, 2'-O-methyluridine, or a combination thereof.

9. The polynucleotide according to claim 7 or 8, further comprising a 5' Cap structure that is Capl or Cap2.

10. The polynucleotide according to claim 9, wherein the 5' Cap structure is Cap1.

11. The polynucleotide according to any one of claims 7 to 10, further comprising Poly(A) of 60 to 160 mers.

12. The polynucleotide according to any one of claims 7 to 11, further comprising a 5' UTR that is a polynucleotide consisting of a nucleotide sequence identical to, or having 90% or greater identity with, a nucleotide sequence set forth in any one of SEQ ID NOs: 1 to 6.

13. The polynucleotide according to any one of claims 7 to 12, further comprising a 3' UTR that is a polynucleotide consisting of a nucleotide sequence identical to, or having 90% or greater identity with, a nucleotide sequence set forth in any one of SEQ ID NOs: 64 to 69.

14. The polynucleotide according to any one of claims 7 to 13, wherein the open reading frame consists of a polynucleotide having a nucleotide sequence set forth in any one of SEQ ID NOs: 114 to 119, 121 to 126, and 198 to 210.

15. The polynucleotide according to any one of claims 7 to 14, wherein the polynucleotide is an mRNA defined by any one of SNG-299, 322 to 325, 327, 328, 331 to 336, 338 to 341, and 345.

16. A pharmaceutical composition comprising the polynucleotide according to any one of claims 1 to 15.

17. The pharmaceutical composition according to claim 16, wherein the pharmaceutical composition is a therapeutic and/or prophylactic agent for HIV infection.
